# EUROPEAN PATENT APPLICATION

(11) **EP 1 005 869 A1**
(43) Date of publication of application: **07.06.2000**
(21) Application number: 98907255.8
(22) Date of filing: 17.03.1998
(51) Int. Cl.: A61K 38/30

(54) **BILE SECRETAGOGUES**

(30) Priority: 24.03.1997 JP 6930997
(71) Applicant: FUJISAWA PHARMACEUTICAL CO., LTD., Osaka-shi Osaka 541-8514 (JP)
(72) Inventor: Ota, Mariko, Kobe-shi Hyougo 655-0046 (JP); KAWAMURA, Ikuo, Hirakata-shi Osaka 573-0093 (JP); MANDA, Toshitaka, Osaka-shi Osaka 533-0021 (JP)
(74) Representative: Gille Hrabal Struck Neidlein Prop Roos
(86) International application number: JP9801109
(87) International publication number: WO9842369

(57) **Abstract**

Bile secretagogues, bile acid secretagogues and remedies for biliary stasis each containing insulin growth factor I or analogs thereof; and a method for stimulating bile secretion, a method for stimulating bile acid secretion and a method for treating biliary stasis each by administering insulin growth factor I or analogs thereof to mammals.

## Description

### TECHNICAL FIELD

This invention relates to a choleretic composition, a bile acid secretion-promoting composition and a therapeutic composition for cholestasis. More particularly, the invention relates to a choleretic composition, a bile acid secretion-promoting composition and a therapeutic composition for cholestasis each comprising insulin-like growth factor (I) or an analog thereof and to a method of promoting secretion of bile, a method of promoting secretion of bile acids, and a method for therapy of cholestasis, each comprising administering insulin-like growth factor I or an analog thereof to mammals.

### BACKGROUND ART

Cholestasis is a symptom of various diseases of the liver. Its etiology, roughly classified, may be iatrogenic (drug-related), viral or congenital. As therapeutic drugs for cholestasis, adrenocortical hormones and ursodeoxycholic acid have heretofore been chiefly used, but adrenocortical hormones have the disadvantage of side effects, while the effect of ursodeoxycholic acid is not fully satisfactory.

### DISCLOSURE OF INVENTION

The inventors of this invention made intensive investigations and found that insulin-like growth factor I (IGF-I) has a potent choleretic action and have developed this instant invention. To be an additional surprise, whereas adrenocortical hormones, such as prednisolone, and ursodeoxycholic acid, all in routine use, exert choleretic actions, IGF-I has both choleretic and bile acid secretion-promoting actions and is expected to provide a new selective therapeutic modality.

This invention, therefore, is directed to a choleretic composition, a bile acid secretion-promoting composition and a therapeutic composition for cholestasis each comprising IGF-I or an analog thereof and further to a method of promoting secretion of bile, a method of promoting secretion of bile acids and a method of treating cholestasis each comprising the use of the corresponding composition.

IGF-I which can be used in this invention includes native IGF-I, a purification product thereof, and IGF-I derived from the human, bovine or other mammalian species by recombinatnt DNA technology (e.g. Kokai Tokkyo Koho S61-1396), a method of peptide synthesis or cell culture. The analog of IGF-I includes muteins of IGF-I as derived by insertion, substitution, deletion or addition of a partial amino acid sequence of IGF-I and chemical modification products such as O-glucosylated IGF-I (WO 90/02198). Such IGF-I species and IGF-I analogs can be produced by the known mutagenic processes or chemical modification methods. Moreover, the specific substance suited for the invention can be selected by a screening assay using a suitable marker such as choleretic activity or bile acid secretion promoting activity. The preferred examples of the IGF-I analog are muteins derived by deletion of 1-5 amino acid residues from the N-terminus as described in WO 89/05822. For the purpose of avoiding unfavorable immunological responses, it is preferable to use human IGF-I or an analog thereof.

A suitable administration method can be selected from among the conventional methods of administration using oral and non-oral (e.g. intravenous, intramuscular, subcutaneous, intraperitoneal, rectal and nasal) routes.

The content of the active substance in the pharmaceutical composition should be just sufficient to produce the expected efficacy and should vary with the patient's age and the route of administration, among other factors. Generally, however, about 1∼100 mg/kg, preferably about 5∼20 mg/kg, as IGF-I can be administered to an adult patient, for instance, in a single dose or in a few divided doses. For the more effective use of the composition of this invention, the conventional drug such as an adrenocortical hormone or ursodeoxycholic acid may be administered concomitantly or in combination.

A suitable dosage form, though dependent on the route of administration, can be selected from among the conventional dosage forms, namely solid dosage forms such as tablets, capsules, powders, granules, fine granules, troches, pills, suppositories, etc., semisolid dosage forms such as an ointment, a cream, etc. , liquid dosage forms such as a solution, a suspension, a syrup, an elixir, etc. and liposomes. Those dosage forms can be manufactured by the established pharmaceutical procedures using suitable carriers, excipients, stabilizers and/or other pharmaceutically acceptable additives. For administration by injection, solid preparations for extemporaneous reconstitution (e.g. freeze-dried products) may also be employed. As specific examples of such dosage forms, the lyophilized preparation described in Kokai Tokkyo Koho H4-208228 and the nasal preparation described in Kokai Tokkyo Koho H5-58877 can be mentioned.

To provide preclinical evidence of the usefulness of this invention, pharmacological data are presented in the following examples.

### Example 1

### Effect of single-dose administration

### (1) Experimental method

With male SD rats (aged 7 weeks) under urethane anesthesia (1.2 g/5 ml/kg), a midline incision was made in the abdominal region. The common bile duct was ligated in an inferior position and a polyethylene tube (PE010, Intramedic, Inc.) was inserted superiorly and ligated in position. The bile was guided extracorporeally and collected. The biliary secretions were measured at 15-min intervals and after the basal secretions were confirmed to be almost steady, IGF-I (3.2 mg/kg) dissolved in saline was administered subcutaneously. The control rats received saline only. The biliary secretions were measured at 15-min intervals up to 180 min after administration and the biliary secretions for 30-min periods were described. The control group was comprised of 4 rats and the IGF-I group of 6 rats.

### (2) Results

In the control group, whereas the basal biliary secretion level before administration was 0.308 ml/30 min (100%), the secretions at 30, 60, 90, 120, 150 and 180 min after administration of saline were 0.310 ml (102%), 0.288 ml (96%), 0.300 ml (99%), 0.298 ml (99%), 0.288 ml (97%) and 0.270 ml (93%), respectively, thus remaining substantially constant up to 150 min, but then declined slightly. In the IGF-I group, whereas the basal secretion level before administration was 0.288 ml/30 min (100%), the secretions at 30, 60, 90, 120, 150 and 180 min after administration were 0.295 ml (101%), 0.312 ml (108%), 0.328 ml (115%), 0.322 ml (112%), 0.337 ml (117%) and 0.308 ml (108%), respectively. After administration of IGF-I, biliary secretions began to increase at 60 mm and continued to increase up to 150 min but began to decline slightly at 180 min.

### Example 2

### Effect of continuous subcutaneous administration

### (1) Experimental method

Using an osmotic pressure pump (Arza, 0.5 µ 1/hr), either IGF-I (320 µ g/day) or saline was continuously administered subcutaneously to male SD rats (aged 5 weeks) for 1 week. For administration, IGF-I (10 mg) was dissolved in 0.375 ml of saline and injected using an osmotic pressure pump. One week after the start of treatment, biliary secretions were measured at 15-min intervals up to 120 min as in Example 1 and the data for 30-min periods were described. The control group and the IGF-I group were comprised of 9 rats each. The concentration of bile acids in each bile sample was determined using a bile acid assay kit (Wako).

### (2) Results

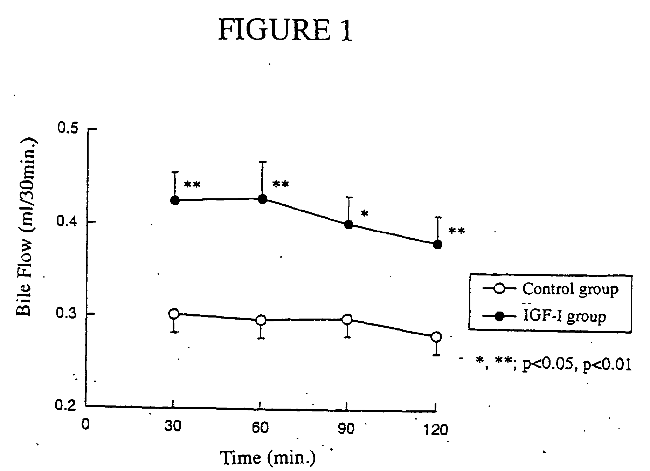

As shown in Fig. 1, the biliary secretions in the control group remained substantially constant at 0.281∼0.302 ml/30 min up to 120 min after the start of measurement. In the IGF-I group, the secretion level was substantially constant at 0.383∼0.429 ml/30 min up to 120 min. The total biliary secretions over the 120-min period amounted to 1.179 ml in the control group vs. 1.641 ml in the IGF-I group, or an overt difference of 39% in favor of IGF-I. The concentration of bile acids in the secreted bile was 18.0±1.9 in the control group vs. 26.4±2.8 µ mol/ml in the IGF-I group, or a difference of 47% in favor of IGF-I. It was thus shown that IGF-I increases not only biliary excretions but also the bile acid concentration of the bile.

### Example 3

### Comparison of IGF-I with prednisolone

### (1) Experimental method

Using IGF-I and prednisolone (PDN) as test drugs and following the experimental protocol of Example 1, biliary secretions were monitored. In addition, the bile acid concentrations of bile samples were determined by the same method as used in Example 2. The bilary secretions and bile acid concentrations at 60∼90 min following administration of the respective drugs corresponding to the peak time of biliary secretion were expressed in percents of the pre-administration baseline values.

### (2) Results

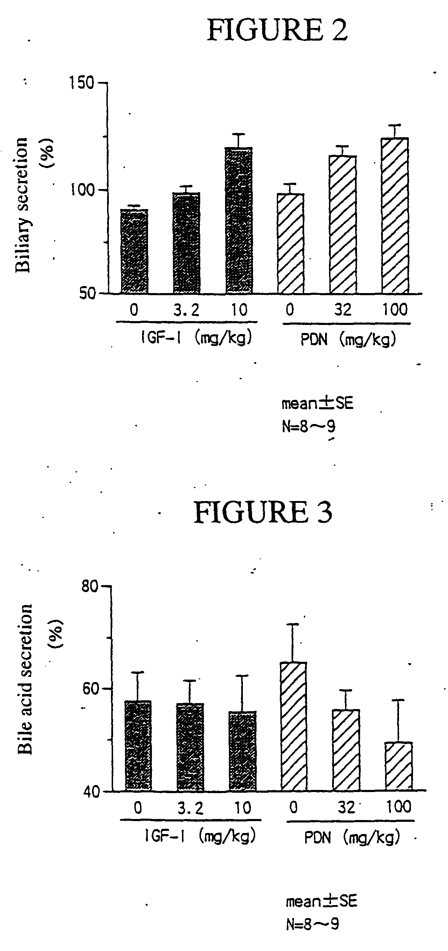

As shown in Fig. 2, both IGF-I and prednisolone increased biliary secretions dose-dependently. Referring to Fig. 3 showing the bile acid concentrations in the IGF-I 10 mg/kg group and the prednisolone 100 mg/kg group, whereas the baseline level was substantially maintained in the IGF-I group, the prednisolone group showed a decline in bile acid concentration.

Those results indicate that both IGF-I and prednisolone have biliary secretion-increasing activity but in the prednisolone group, an increase in biliary secretions was accompanied by a decrease in bile acid concentration resulting in no remarkable change in the absolute amount of bile acids secreted. This is in contrast with the IGF-I group in which the concentration of bile acids remained constant despite increased biliary secretions, resulting in an increase in the absolute amount of bile acids secreted. The above findings suggest that the two drugs are dissimilar in the mechanism of choleretic action and that this invention offers a new alternative therapeutic modality for refractory diseases such as biliary atresia.

### BRIEF DESCRIPTION OF THE DRAWINGS

Fig. 1 is a diagrammatic representation of the bile flows in the solvent control group and IGF-I group.
Fig. 2 is a histogram showing the relationships of dosage to biliary secretions in the IGF-I group and prednisolone group.
Fig. 3 is a histogram showing the relationships of dosage to bile acid concentration in the IGF-I group and prednisolone group.

## Claims

1. A choleretic composition comprising insulin-like growth factor I or an analog thereof.

2. A bile acid secretion-promoting composition comprising insulin-like growth factor I or an analog thereof.

3. A therapeutic composition for cholestasis which comprises insulin-like growth factor I or an analog thereof.

4. A method of promoting secretion of bile which comprises administering insulin-like growth factor I or an analog thereof to a mammal.

5. A method of promoting secretion of bile acids which comprises administering insulin-like growth factor I or an analog thereof to a mammal.

6. A method of treating cholestasis which comprises administering insulin-like growth factor I or an analog thereof to a mammal.

7. Use of insulin-like growth factor I or an analog thereof for promoting secretion of bile.

8. Use of insulin-like growth factor I or an analog thereof for promoting secretion of bile acids.

9. Use of insulin-like growth factor I or an analog thereof for the therapy of cholestasis.
